# EUROPÄISCHE PATENTSCHRIFT

(11) **EP 2 445 546 B1**
(45) Veröffentlichungstag und Bekanntmachung des Hinweises auf die Patenterteilung: **01.10.2014**
(21) Anmeldenummer: 10725618.2
(22) Anmeldetag: 16.06.2010
(51) Int. Cl.: A61M 1/36

(54) **KAMMER FÜR EIN BLUTBEHANDLUNGSSYSTEM, BLUTSCHLAUCHSYSTEM SOWIE BLUTBEHANDLUNGSSYSTEM**
CHAMBER FOR A BLOOD TREATMENT SYSTEM, BLOOD TUBING SYSTEM, AND BLOOD TREATMENT SYSTEM
CHAMBRE POUR SYSTÈME DE TRAITEMENT SANGUIN, SYSTÈME DE TUBES SOUPLES POUR LE SANG, ET SYSTÈME DE TRAITEMENT SANGUIN

(30) Priorität: 24.06.2009 DE 102009030283
(43) Veröffentlichungstag der Anmeldung: 02.05.2012
(73) Patentinhaber: Fresenius Medical Care Deutschland GmbH, 61352 Bad Homburg (DE)
(72) Erfinder: SPICKERMANN, Reiner, 97535 Wasserlosen-Burghausen (DE); WIESEN, Gerhard, 61352 Bad Homburg (DE)
(74) Vertreter: Herrmann, Uwe
(86) Internationale Anmeldenummer: PCT/EP2010/003596
(87) Internationale Veröffentlichungsnummer: WO 2010/149298

(56) Entgegenhaltungen:
- WO-A1-2004/000391
- WO-A2-2007/050211
- DE-A1- 3 202 582
- US-A- 5 421 815
- US-A- 5 858 239

## Beschreibung

Die vorliegende Erfindung betrifft eine Kammer für ein Blutbehandlungssystem mit einem Bluteinlass und einem Blutauslass, einem bezogen auf die Lage der Kammer im Betriebszustand kopfseitig angeordneten Filterelement zur Luftabscheidung, das mittels einer weiteren Flüssigkeit vom in der Kammer im Betriebszustand befindlichen Blut entkoppelbar ist, ein Blutschlauchsystem sowie ein Blutbehandlungssystem.

Stromabwärts eines Dialysierfilters befindet sich bei bekannten Hämodialysesystemen eine venöse Tropfkammer, die Bestandteil des extrakorporalen Blutkreislaufs ist. Sie sorgt an dieser Stelle für eine blasenfreie Reinfusion des dialysierten Patientenblutes. Üblicherweise ist die venöse Tropfkammer nicht vollständig gefüllt. Somit kommt es hier zu einem nachteiligen Blut-Luft-Kontakt. Zur Vermeidung von einer Infusion von Blutgerinnseln ist die venöse Tropfkammer mit einem Gerinselfänger ausgestattet, welcher in der Regel als Sieb ausgeführt ist.

Aus dem Stand der Technik sind bereits mehrere Beispiele für Tropfkammern bekannt.

So zeigt die DE 32 02 582 A1 eine Tropfkammer, bei der eintropfendes Blut nicht direkt in den Blutspiegel fällt, sondern auf eine schräge Innenwand, um eine Schaumbildung und damit die Gefahr einer möglichen Hämolyse zu minimieren.

Die US 5,330,425 beschreibt mehrere blasgeformte Tropfkammern für die Dialysebehandlung mit einer speziell lokalisierten Zuspritzstelle.

Die US 3,834,386 beschreibt eine Tropfkammer mit einem in den Deckel der Tropfkammer integrierten Septum.

Der Bluteinlass in die Kammer erfolgt im Stand der Technik häufig über einen entsprechenden Port, dessen Ende über dem Blutspiegel liegt. Es erfolgt also ein Abtropfen des Blutes in die Kammer, was die Gefahr von Mikroblasenbildung birgt, wobei derartige Mikroblasen nicht abgeschieden und ungehindert zum Patienten zurückgeführt werden können. Außerdem können diese Mikroblasen Hämolyse verursachen.

Eine Standardtropfkammer verfügt meist weiterhin über einen Zuspritzport mit einem hydrophoben Septum und über eine Belüftungsleitung, die mit einer hydrophoben Membran, insbesondere einem sogenannten Transducer-Protector (TDP), versehen ist. Über ein 3-Wege-Ventil im Inneren der Maschine kann die Belüftungsleitung einerseits auf einen Druckmessaufnehmer zur Kontrolle des Druckes in der venösen Tropfkammer geführt werden, oder aber andererseits über einen Druckminderer die Tropfkammer belüften, wie dies beispielsweise beim initialen Befüllen des Schlauchsystems bzw. des extrakorporalen Blutkreislaufes der Fall ist.

Weiter ist aus der WO 2007/050211 A2 eine venöse Tropfkammer bekannt, bei der der Bluteinlassport und der Blutauslassport im Boden der Kammer angeordnet sind, wobei sich zwischen Bluteinlassport und Blutauslassport eine Trennwand befindet. Im Deckel der Kammer ist eine Hydrophobmembran eingelassen, um die Kammer zu belüften. Die Kammer ist zu Beginn komplett mit Kochsalzlösung befüllt, wobei die Befüllung über einen Bluteinlassport erfolgt. Wenn im Dialysebetrieb Blut in die Kammer strömt, wird die verdrängte Kochsalzlösung dem Patienten zugegeben. Es verbleibt eine gewisse Menge an Kochsalzlösung in der Kammer, um die Hydrophobmembran vom Blutspiegel zu entkoppeln. Das Handling einer derartigen Tropfkammer ist mit nicht unerheblichem Aufwand und erheblicher Sorgfalt verbunden, da Sorge getragen werden muss, dass ausreichend Kochsalzlösung in der Kammer verbleibt, um die Hydrophobmembran vom Blutspiegel zu entkoppeln und eine korrekte Funktionsfähigkeit der Tropfkammer zu gewährleisten.

Es ist daher die Aufgabe der vorliegenden Erfindung, eine Kammer für ein Blutbehandlungssystem der eingangs genannten Art in vorteilhafter Weise weiterzubilden, insbesondere dahingehend, dass eine venöse Tropfkammer mit minimiertem Blut-Luft-Kontakt geschaffen wird, die zuverlässig auch die Bildung von Mikroblasen verhindert und darüber hinaus einfach und sicher betrieben werden kann.

Diese Aufgabe wird erfindungsgemäß gelöst durch eine Kammer für ein Blutbehandlungssystem mit den Merkmalen des Anspruchs 1. Danach ist vorgesehen, dass eine Kammer für ein Blutbehandlungssystem einen Bluteinlass und einen Blutauslass, sowie ein bezogen auf die Lage der Kammer im Betriebszustand kopfseitig angeordnetes Filterelement zur Luftabscheidung aufweist, das mittels einer weiteren Flüssigkeit von in der Kammer im Betriebszustand befindlichen Blut entkoppelbar ist. Weiter ist vorgesehen, dass der Bluteinlass kopfseitig angeordnet ist. Durch die kopfseitige Anordnung des Bluteinlasses ergibt sich der Vorteil, dass die einfache Handhabbarkeit bei der erfindungsgemäßen Kammer, wie sie auch bereits bei den bisherigen Tropfkammern der Fall war, beibehalten werden kann. Denn der kopfseitige Bluteinlass erlaubt ein einfaches Befüllen der Kammer mit Blut. Dies ist insbesondere beim initialen Entlüften der Tropfkammer von Vorteil. Durch das kopfseitig angeordnete Filterelement zur Luftabscheidung wird die Luftabscheidung nicht mehr über einen Transducer-Protector geleistet, sondern direkt über eine Filtermembran, die mittels einer weiteren Flüssigkeit vom Blut entkoppelt ist, so dass ein sogenanntes Clotting von Blut am Filterelement verhindert wird.

Die Kammer weist zudem vorteilhafterweise keine Belüftungsleitung mehr auf. Folglich kann es auch nicht zur einer Benetzung oder einem Durchbruch des vorteilhafterweise nicht mehr vorhandenen Transducer-Protectors (TDP) kommen, wodurch die Gefahr, z. B. die angeschlossene Dialysemaschine zu kontaminieren, ausgeschlossen ist.

Somit können mehrere Probleme des gegenwärtigen extrakorporalen Kreislaufs gelöst werden. Es ist möglich, eine Umstellung der Druckmessung auf eine nicht invasive Messung mittels beispielsweise eines Druckdomes vorzunehmen, wobei es zu einem Wegfall der Transducer-Protector-Probleme kommt. Weiter treten auch die mit dem Transducer-Protector verbundenen maschinenseitigen Kontaminationsfolgen nicht mehr auf, da beispielsweise ein Austausch eines Druckaufnehmers nach einem Blutkontakt nicht mehr erfolgen muss. Nachdem sich im Betriebszustand der Kammer keine Luft befindet, da vorzugsweise die untere Hälfte vollständig mit Blut gefüllt ist und sich oberhalb des Blutspiegels die weitere Flüssigkeit befindet, die das Filterelement vom in der Kammer im Betriebszustand befindlichen Blut entkoppelt, gibt es keinen unmittelbaren Blut-Luft-Kontakt und keine Stagnationsbereiche in der Kammer. Dadurch kann der Bedarf an Gerinnungshemmern deutlich reduziert werden. Darüber hinaus wird die Bildung von Mikroblasen in der venösen Kammer verhindert.

Es kann vorgesehen sein, dass der Blutauslass bezogen auf die Lage der Kammer im Betriebszustand bodenseitig in der Kammer angeordnet ist.

Des Weiteren ist vorgesehen, dass eine Kammer für ein Blutbehandlungssystem einen Bluteinlass und einen Blutauslass, sowie ein bezogen auf die Lage der Kammer im Betriebszustand kopfseitig angeordnetes Filterelement zur Luftabscheidung aufweist, das mittels einer weiteren Flüssigkeit vom in der Kammer im Betriebszustand befindlichen Blut entkoppelbar ist, wobei die weitere Flüssigkeit mittels eines Zuführportes zuführbar ist. Dadurch ergibt sich der Vorteil, dass die weitere Flüssigkeit zur Entkopplung des Filterelements vom in der Kammer im Betriebszustand befindlichen Blut während des Betriebes zuführbar ist. So kann gegebenenfalls Flüssigkeit nachgefüllt werden, wenn ein Teil der entkoppelnden Flüssigkeit sich mit dem Blut in der Kammer vermischt und somit im Betrieb die Kammer durch den Blutauslass verlassen sollte.

Es kann vorgesehen sein, dass die Kammer mit den Merkmalen des Anspruchs 3 die kennzeichnenden Merkmale der Ansprüche 1 oder 2 aufweist.

Weiter kann vorgesehen sein, dass die Kammer mit den Merkmalen der Ansprüche 1 oder 2 die kennzeichnenden Merkmale des Anspruch 3 aufweist.

Ferner ist denkbar, dass die weitere Flüssigkeit kontinuierlich zuführbar ist oder in vordefinierten Zeitabständen zuführbar ist und/oder dass das Filterelement eine hydrophobe Filtermembran umfasst oder durch diese ausgebildet ist. Dadurch ergibt sich der Vorteil, dass während des Betriebes sichergestellt ist, dass sich das Filterelement nicht zusetzt. Insbesondere ist bei der kontinuierlichen Flüssigkeitszuführung von Vorteil, dass eine kontinuierliche Luftabscheidung aufgrund der kontinuierlich angeströmten Hydrophobmembran sichergestellt wird. Es wird hierdurch wirksam verhindert, dass sich Blutbestandteile an der Hydrophobmembran ansetzen können. Der gleiche Effekt kann auch dadurch erzielt werden, dass die weitere Flüssigkeit in vordefinierten Zeitabständen, etwa getaktet zugeführt wird. Eine Luftabscheidung aus dem Blut kann dann an der Grenzfläche zwischen Blut und der weiteren Flüssigkeit erfolgen. Die aus dem Blut aufsteigende Luft tritt über die Grenzfläche zwischen Blut und der weiteren Flüssigkeit über und steigt sodann nach oben. Dort kann sie über den Hydrophobfilter entweichen. An der Flüssigkeitsgrenze zwischen Blut und der weiteren Flüssigkeit kann es zu einer Vermengung der beiden Stoffe kommen, was beispielsweise einer Bolusgabe bei einer Hämofiltration bzw. Hämodiafiltration gleich kommt. Um vorteilhafterweise den Substituatspiegel bzw. den Flüssigkeitsspiegel der weiteren Flüssigkeit um die Hydrophobmembran aufrechtzuerhalten, muss das Substituat permanent nachgefüllt wird. Dies gelingt vorzugsweise über die Einstellung eines Substituatflusses über den Zuführport und entsprechende Mittel zur Substituatsteuerung z. B. einer Dialysemaschine. Das eingebrachte Substituat kommt hierbei nicht mit Luft in Kontakt, im Gegensatz zu Tropfkammern, bei denen das Substituat über einen Infusionsport eingetropft wird. Das vermeidet die Bildung von Mikroblasen und deren Infusion in den Patienten. Die Kammer ist beispielsweise auch für die Hämodialyse geeignet, wenn die eingebrachte Substituatmenge klein ist.

Darüber hinaus kann vorgesehen sein, dass der Zuführport ein Rückschlagventil und/oder ein Rückstromverhinderungsmittel aufweist. Hierdurch ergibt sich der Vorteil, dass wirksam ein Einströmen von Blut in den Zuführport verhindert werden kann.

Des Weiteren kann vorgesehen sein, dass die Kammer einen Infusionsport und/oder vor dem Blutauslass ein Siebmittel aufweist. Das Siebmittel kann beispielsweise ein sogenannter Gerinselfänger sein, Durch den Infusionsport ist es einfach möglich, eine oder mehrere Infusionen an die Kammer anzuschließen.

Weiter ist denkbar, dass die Kammer einen im Wesentlichen zylindrischen Grundkörper aufweist und die Kammer in ihrem oberen Ende derart exzentrisch und/oder aufgeweitet ausgebildet ist, dass das über den Bluteinlass in die Kammer eintretende Blut auf eine dadurch gebildete Schrägwandung auftropfbar ist, wobei der Bluteinlass exzentrisch und parallel zur Mittelachse der Kammer angeordnet ist, so dass das Blut außermittig der Kammer zuführbar ist. Hierdurch ergibt sich der Vorteil, dass das Auftreten von Mikrobläschen weiter verhindert wird, da zum Einen beim initialen Befüllen eine Schaumbildung wirksam verhindert werden kann. Zum Anderen wird beim initialen Befüllen hierdurch der Weg durch die Luft eines Bluttropfens bis zum Auftreffen auf die Wandung verringert.

Insbesondere kann vorgesehen sein, dass der Bluteinlass im Betriebszustand bei gefüllter Kammer unterhalb des sich im Betrieb einstellenden Blutspiegels befindet. Das heißt, dass die Auslassöffnung des Bluteinlasses sich unterhalb des Blutspiegels in der Kammer befindet und das der Kammer zugeführte Blut direkt und ohne Umwege oder Luftkontakt in das Blutkompartiment in der Kammer eintreten kann. Des Weiteren kann vorgesehen sein, dass der Auslass des Zuführportes für das Substituat unmittelbar neben dem kopfseitigen Filterelement angeordnet ist. Dadurch ergibt sich der Vorteil, dass eine einfache und sichere Umströmung des Filterelementes gewährleistet werden kann.

Außerdem kann vorgesehen sein, dass die Kammer eine venöse Tropfkammer eines Blutschlauchsystems, insbesondere eines Blutschlauchsystems für die Dialyse, ist.

Ferner ist denkbar, dass die Flüssigkeit, die über den Zuführport zuführbar ist, eine gefilterte und pyrogenfreie Flüssigkeit, insbesondere pyrogenfreies und gefiltertes Dialysat ist und/oder eine Kochsalzlösung ist.

Des Weiteren ist vorgesehen, dass ein Blutschlauchsystem mit wenigstens einer Kammer nach einem der Ansprüche 1 bis 11 versehen ist.

Weiter ist vorgesehen, dass ein Blutbehandlungssystem mit wenigstens einer Kammer nach einem der Ansprüche 1 bis 11 oder einem Blutschlauchsystem nach Anspruch 12 versehen ist.

Es kann vorgesehen sein, dass das Blutbehandlungssystem eine Dialysemaschine ist und dass die Flüssigkeit, die über den Zuführport zuführbar ist, gefiltertes und pyrogenfreies Dialysat ist, dass durch die Dialysemaschine bereitgestellt und/oder aufbereitet wird.

Weitere Einzelheiten und Vorteile sollen nun anhand eines in der Zeichnung dargestellten Ausführungsbeispiels näher erläutert werden. Es zeigen:
- Figur 1:: eine schematische Darstellung einer erfindungsgemäßen Kammer für ein Blutbehandlungssystem und
- Figur 2:: eine weitere schematische Darstellung der in Figur 1 gezeigten Kammer.

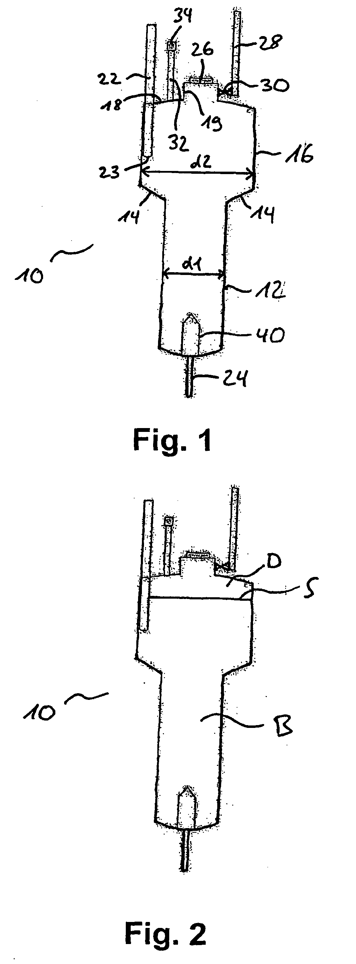

Figur 1 zeigt in schematischer Darstellung die erfindungsgemäße Kammer 10, die als venöse Tropfkammer 10 eines venösen Teils eines extrakorporalen Blutschlauchsystems für eine Dialysemaschine ausgebildet ist. Die venöse Tropfkammer 10 weist dabei im unteren Bereich einen zylinderförmigen Grundkörper 12 mit einem geringeren Durchmesser d1 auf, als dies im oberen Bereich 16, der ebenfalls zylinderförmig ausgebildet ist, der Fall ist. Die Bereiche 12 und 16 werden dabei durch eine schräg stehende umlaufende Schrägwand 14 verbunden. Die Kammer 10 ist dadurch derart in ihrem oberen Ende aufgeweitet ausgebildet, dass das über den Bluteinlass 22 in die Kammer 10 eintretende Blut auf eine Schrägwandung 14 auftropfbar ist.

Der Bluteinlass 22 ist dabei exzentrisch und parallel zur Mittelachse der Kammer 10 angeordnet, so dass das Blut außermittig der Kammer zuführbar ist. Der Bluteinlass 22 ist dabei ein starres Rohrstück 22, dessen Auslassöffnung, wie in Figur 2 gezeigt, sich im Betriebszustand unterhalb des sich in der Kammer 10 einstellenden Blutspiegels B befindet. Das Bluteinlassrohrstück 22 liegt dabei an der Innenseite der Wandung des oberen Bereichs 16 an, der einen Durchmesser d2 aufweist. Der Durchmesser d2 ist dabei größer als der Durchmesser d1, wodurch sichergestellt wird, dass das aus der Austrittsöffnung 23 initial beim Befüllen der Kammer 10 austretende Blut auf die Schrägwandung 14 aufgetropft wird und nicht auf das sich bereits im unteren Bereich 12 befindliche Blut. Das dialysierte, in die Kammer 10 eintretende Blut verläßt die Kammer 10 nach dem Durchtritt durch den Gerinselfänger 40, der vor dem Blutauslass 24 bodenseitig angeordnet ist.

Im Kammerdach 18 ist zentral eine domartige Ausformung 19 vorgesehen, in deren oberen Teil das als Hydrophobmembran 26 ausgeführte Filterelement 26 angeordnet ist. Über das Filterelement 26 wird die Luftabscheidung aus der Kammer erreicht. Gegebenenfalls kann dieses Filterelement 26 eine Doppelmembran sein oder einen Zusatzfilter wie z. B. Porex aufweisen, was beispielsweise bei Kondensationseffekten von Vorteil sein kann.

In bevorzugter Ausgestaltung der vorliegenden Erfindung befindet sich das Filterelement 26 oberhalb der Austrittsöffnung 23 und besonders bevorzugt an oberster Stelle der Kammer 10.

Um die kontinuierliche Luftabscheidung über die gesamte Dialysedauer zu gewährleisten, wird die Hydrophobmembran 26 über die gesamte Dialysedauer mit Dialysierflüssigkeit angespült, die über den Zuführport 28 mit dem Rückschlagventil 30 in die Kammer zugeführt wird. Das Rückschlagventil 30 verhindert, dass Blut in die Zuführleitung 28 eintreten kann. Die Zuführleitung 28 ist mit der Dialysatleitung der Dialysemaschine verbunden, so dass das in der Dialysemaschine aufbereitete, pyrogenfreie und gefilterte Dialysat über den Zuführport 28 während der Dialysedauer kontinuierlich in die Kammer zugeführt werden kann, so dass das Filterelement 26 stetig gespült ist.

Es stellt sich dabei die in Figur 2 gezeigte Flüssigkeitsverteilung ein, wobei das im Blut B befindliche Gas, insbesondere Luft über die Trennfläche S in das Dialysat D bzw. Substituat D aufsteigt und von dort zum Luftabscheider 26 gelangt.

Weiter weist die Kammer einen Infusionsport 32 auf, der über ein Septum 34 oder einen Luer-Konnektor 34 verfügt.

## Patentansprüche

1. Kammer (10) für ein Blutbehandlungssystem mit einem Bluteinlass (22) und einem Blutauslass (24), wobei ein bezogen auf die Lage der Kammer im Betriebszustand kopfseitig angeordnetes Filterelement (26) zur Luftabscheidung vorgesehen ist, das mittels einer weiteren Flüssigkeit (D) vom in der Kammer (10) im Betriebszustand befindlichen Blut (B) entkoppelbar ist,
**dadurch gekennzeichnet,**
**dass** der Bluteinlass kopfseitig angeordnet ist und dass die Kammer einen Zuführport (28) aufweist, mittels dessen die weitere Flüssigkeit (D) zuführbar ist, wobei die weitere Flüssigkeit (D) durch den Zuführport (28) kontinuierlich zuführbar ist oder in vordefinierten Zeitabständen zuführbar ist, um während des Betriebes ein Zusetzen des Filterelementes (26) zu verhindern.

2. Kammer (10) nach Anspruch 1, **dadurch gekennzeichnet, dass** der Blutauslass (24) bezogen auf die Lage der Kammer (10) im Betriebszustand bodenseitig in der Kammer (10) angeordnet ist.

3. Kammer (10) nach Anspruch 1 oder 2, **dadurch gekennzeichnet, dass** das Filterelement (26) eine hydrophobe Filtermembran umfasst oder durch diese ausgebildet ist.

4. Kammer (10) nach einem der vorhergehenden Ansprüche, **dadurch gekennzeichnet, dass** der Zuführport (28) ein Rückschlagventil (30) und/oder ein Rückstromverhinderungsmittel (30) aufweist.

5. Kammer (10) nach einem der vorhergehenden Ansprüche, **dadurch gekennzeichnet, dass** die Kammer (10) einen Infusionsport (22) aufweist.

6. Kammer (10) nach einem der vorhergehenden Ansprüche, **dadurch gekennzeichnet, dass** die Kammer (10) vor dem Blutauslass (24) ein Siebmittel (40) aufweist.

7. Kammer (10) nach einem der vorhergehenden Ansprüche, **dadurch gekennzeichnet, dass** die Kammer (10) einen im Wesentlichen zylindrischen Grundkörper aufweist und die Kammer (10) in ihrem oberen Ende derart exzentrisch und/oder aufgeweitet ausgebildet ist, dass das über den Bluteinlass (22) in die Kammer (10) eintretende Blut auf eine dadurch gebildete Schrägwandung (14) auftropfbar ist, wobei der Bluteinlass (22) exzentrisch und parallel zur Mittelachse der Kammer (10) angeordnet ist, so dass das Blut außermittig der Kammer (10) zuführbar ist.

8. Kammer (10) nach einem der vorhergehenden Ansprüche, **dadurch gekennzeichnet, dass** die Auslassöffnung (23) des Bluteinlasses (22) sich im Betrieb unterhalb des sich in der Kammer (10) einstellenden Blutspiegels (S) befindet.

9. Kammer (10) nach einem der vorhergehenden Ansprüche, **dadurch gekennzeichnet, dass** der Auslass des Zuführportes (28) unmittelbar neben dem kopfseitigen Filterelement (26) angeordnet ist.

10. Kammer nach einem der vorhergehenden Ansprüche, **dadurch gekennzeichnet, dass** die Kammer (10) eine venöse Tropfkammer (10) eines Blutschlauchsystems, insbesondere eines Blutschlauchsystems für die Dialyse, ist.

11. Kammer (10) nach einem der vorhergehenden Ansprüche, **dadurch gekennzeichnet, dass** die Flüssigkeit (D), die über den Zuführport (28) zuführbar ist, eine gefilterte und pyrogenfreie Flüssigkeit, insbesondere pyrogenfreies und gefiltertes Dialysat ist und/oder eine Kochsalzlösung ist.

12. Blutschlauchsystem mit wenigstens einer Kammer (10) nach einem der Ansprüche 1 bis 11.

13. Blutbehandlungssystem mit wenigstens einer Kammer (10) nach einem der Ansprüche 1 bis 11 oder mit einem Blutschlauchsystems nach Anspruch 12.

14. Blutbehandlungssystem nach Anspruch 13, **dadurch gekennzeichnet, dass** das Blutbehandlungssystem eine Dialysemaschine ist und dass die Flüssigkeit (D), die über den Zuführport (28) zuführbar ist, gefiltertes und pyrogenfreies Dialysat ist, dass durch die Dialysemaschine bereitgestellt und/oder aufbereitet wird.

## Claims

1. A chamber (10) for a blood treatment system having a blood inlet (22) and a blood outlet (24), wherein a filter element (26) for air separation is provided which is arranged at the head side with respect to the position of the chamber in the operating state and which can be decoupled from the blood (B) present in the chamber (10) in the operating state by a further liquid (D),
**characterized in that**
the blood inlet is arranged at the head side and **in that** the chamber has a supply port (28) by means of which the further fluid (D) can be supplied, wherein the further liquid (D) can be supplied continuously by the supply port (28) or can be supplied at predefined time intervals to prevent a clogging of the filter element (26) during operation.

2. A chamber (10) in accordance with claim 1, **characterized in that** the blood outlet (24) is arranged in the chamber (10) at the base side with respect to the position of the chamber (10) in the operating state.

3. A chamber (10) in accordance with claim 1 or 2, **characterized in that** the filter element (26) includes a hydrophobic filter membrane or is formed by it.

4. A chamber (10) in accordance with one of the preceding claims, **characterized in that** the supply port (28) has a check valve (30) and/or a means preventing backflow (30).

5. A chamber (10) in accordance with one of the preceding claims, **characterized in that** the chamber (10) has an infusion port (22).

6. A chamber (10) in accordance with one of the preceding claims, **characterized in that** the chamber (10) has a screen means (40) in front of the blood outlet (24).

7. A chamber (10) in accordance with one of the preceding claims, **characterized in that** the chamber (10) has a substantially cylindrical base body and the chamber (10) is formed eccentrically and/or in a flared manner in its upper end such that the blood entering into the chamber (10) via the blood inlet (22) can be dripped onto an oblique wall (14) formed thereby, with the blood inlet (22) being arranged eccentrically and parallel to the center axis of the chamber (10) so that the blood can be supplied to the chamber (10) in an off-center manner.

8. A chamber (10) in accordance with one of the preceding claims, **characterized in that** the outlet opening (23) of the blood inlet (22) is located beneath the blood level (S) adopted in the chamber (10) in operation.

9. A chamber (10) in accordance with one of the preceding claims, **characterized in that** the outlet of the supply port (28) is arranged directly next to the filter element (26) at the head side.

10. A chamber in accordance with one of the preceding claims, **characterized in that** the chamber (10) is a venous drip chamber (10) of a blood hose system, in particular of a blood hose system for dialysis.

11. A chamber (10) in accordance with one of the preceding claims, **characterized in that** the liquid (D) which can be supplied via the supply port (28) is a filtered and pyrogen-free liquid, in particular a pyrogen-free and filtered dialysate and/or is a saline solution.

12. A blood hose system having at least one chamber (10) in accordance with one of the claims 1 to 11.

13. A blood treatment system having at least one chamber (10) in accordance with one of the claims 1 to 11 or having a blood hose system in accordance with claim 12.

14. A blood treatment system in accordance with claim 13, **characterized in that** the blood treatment system is a dialysis machine; and **in that** the liquid (D) which can be supplied via the supply port (28) is a filtered and pyrogen-free dialysate which is provided and/or treated by the dialysis machine.

## Revendications

1. Chambre (10) pour système de traitement sanguin, comprenant une entrée de sang (22) et une sortie de sang (24), un élément filtrant (26) disposé côté tête par rapport à la position de la chambre à l'état de fonctionnement étant prévu pour le dégagement d'air, l'élément filtrant pouvant être découplé du sang (B) qui se trouve dans la chambre (10) à l'état de fonctionnement au moyen d'un autre liquide (D),
**caractérisée en ce que**
l'entrée de sang est disposée côté tête et **en ce que** la chambre comporte un port d'alimentation (28), au moyen duquel l'autre liquide (D) peut être alimenté, l'autre liquide (D) pouvant être alimenté en continu ou à intervalles prédéfinis par le port d'alimentation (28) pour empêcher un engorgement de l'élément filtrant (26) pendant le fonctionnement.

2. Chambre (10) selon la revendication 1, **caractérisée en ce que** la sortie de sang (24) est disposée côté fond dans la chambre (10) par rapport à la position de la chambre (10) à l'état de fonctionnement.

3. Chambre (10) selon la revendication 1 ou 2, **caractérisée en ce que** l'élément filtrant (26) comprend une membrane filtrante hydrophobe ou est formé par celle-ci.

4. Chambre (10) selon l'une des revendications précédentes, **caractérisée en ce que** le port d'alimentation (28) comporte un clapet anti-retour (30) et/ou un moyen anti-reflux (30).

5. Chambre (10) selon l'une des revendications précédentes, **caractérisée en ce que** la chambre (10) comporte un port de perfusion (22).

6. Chambre (10) selon l'une des revendications précédentes, **caractérisée en ce que** la chambre (10) comporte un moyen de tamisage (40) avant la sortie de sang (24).

7. Chambre (10) selon l'une des revendications précédentes, **caractérisée en ce que** la chambre (10) comporte un corps de base essentiellement cylindrique et la chambre (10) est formée à son extrémité supérieure de manière tellement excentrée et/ou élargie que le sang entrant dans la chambre (10) par l'entrée de sang (22) peut être instillé sur une paroi oblique (14) formée de cette manière, l'entrée de sang (22) étant disposée excentrée par rapport à l'axe central de la chambre (10) et parallèle à celui-ci de sorte que le sang peut être alimenté de manière excentrée par rapport à la chambre (10).

8. Chambre (10) selon l'une des revendications précédentes, **caractérisée en ce que** l'orifice de sortie (23) de l'entrée de sang (22) se trouve pendant le fonctionnement sous le niveau de sang (S) s'établissant dans la chambre (10).

9. Chambre (10) selon l'une des revendications précédentes, **caractérisée en ce que** la sortie du port d'alimentation (28) est disposée directement à côté de l'élément filtrant (26) côté tête.

10. Chambre selon l'une des revendications précédentes, **caractérisée en ce que** la chambre (10) est une chambre compte-gouttes veineuse (10) d'un système de tubes souples pour le sang, notamment d'un système de tubes souples pour le sang destiné à la dialyse.

11. Chambre (10) selon l'une des revendications précédentes, **caractérisée en ce que** le liquide (D) qui peut être alimenté via le port d'alimentation (28) est un liquide filtré et apyrogène, notamment du dialysat apyrogène et filtré, et/ou est une solution physiologique.

12. Système de tubes souples pour le sang comprenant au moins une chambre (10) selon l'une des revendications 1 à 11.

13. Système de traitement sanguin comprenant au moins une chambre (10) selon l'une des revendications 1 à 11, ou comprenant un système de tubes souples pour le sang selon la revendication 12.

14. Système de traitement sanguin selon la revendication 13, **caractérisé en ce que** le système de traitement sanguin est une machine de dialyse et **en ce que** le liquide (D) qui peut être alimenté via le port d'alimentation (28) est du dialysat filtré et apyrogène qui est mis à disposition et/ou traité par la machine de dialyse.
